# EUROPEAN PATENT APPLICATION

(11) **EP 2 530 462 A1**
(43) Date of publication of application: **05.12.2012**
(21) Application number: 12275088.8
(22) Date of filing: 06.06.2012
(51) Int. Cl.: G01N 33/50, G01N 33/574, A61K 39/395

(54) **Methods and kits for predicting tumor responses to antibodies against epidermal growth factor receptor (EGFR) and uses thereof**

(30) Priority: 03.06.2011 US 201161493252 P
(71) Applicant: Merrimack Pharmaceuticals, Inc., Cambridge, MA 02139 (US)
(72) Inventor: Bukhalid, Raghida, Melrose, MA Massachusetts 02176 (US); Nielsen, Ulrik, Quincy, MA Massachusetts 02169 (US); Werner, Shannon, Belmont, MA Massachusetts 02478 (US); Kearns, Jeffrey David, Arlington, MA Massachusetts 02474-6902 (US)
(74) Representative: Tuxworth, Pamela M.

(57) **Abstract**

Disclosed are pharmaceutical preparations for, and methods for determining, appropriate and effective treatment with therapeutic agents comprising a single species of anti-EGFR monoclonal antibody or therapeutic agents comprising a plurality of species of such antibodies, as well as kits useful for making such determinations.

## Description

### Background

The epidermal growth factor receptor (EGFR) is a cell surface transmembrane receptor of the HER/ErbB receptor family that transmits signals (including mitogenic signals that drive cell proliferation) to the interior of a cell when activated, typically by the binding of any of a number of extracellular ligands such as epidermal growth factor (EGF). EGFR ligands vary in their affinity for EGFR and are categorized as either high- or low-affinity ligands. It is thought that the high- and low-affinity interactions between EGFR and its ligands activate different signaling pathways. This signal transmission occurs through a cascade of intracellular events beginning with protein phosphorylation mediated by receptor tyrosine kinase activity. EGFR has proven a responsive target for anti-proliferative (*e*.*g*., anti-cancer) drugs, including "small molecule" tyrosine kinase inhibitor drugs (typically no larger than 700-900 AMU) that may be orally administered as well as monoclonal antibody based drugs that specifically bind to the extracellular domain of EGFR.

EGFR-targeted monoclonal antibodies are not always effective against EGFR-expressing tumors. One approach taken with the aim of improving anti-EGFR antibody efficacy has been to develop mixtures of anti-EGFR monoclonal antibodies (*i.e.*, oligoclonal antibodies) targeted to different sites (epitopes) on to the extracellular domain of EGFR. See, *e*.*g*., PCT Int. Pub. No. W02011/140254 and US Patent No. 7,887,805. These developments have created a need to enable the identification of cancer patients whose tumors have characteristics rendering them unresponsive to monoclonal anti-EGFR antibodies so that such patients may receive effective treatment via administration of oligoclonal anti-EGFR antibodies. The present disclosure answers this need and provides other benefits.

### Summary

Provided herein are theranostic methods for predicting responsiveness of cancer cells that express EGFR to therapeutic agents comprising anti-EGFR antibodies, and antibody preparations for use for treating patients having tumors predicted to be responsive to treatment with such therapeutic agents. Anti-EGFR antibodies such as those monoclonal and oligoclonal antibodies described in PCT Int. Pub. No. WO2011/140254 and in US Patent No. 7,887,805 (the "Oligoclonal Publications"), as well as oligoclonal mixtures of such monoclonal antibodies in combination with other anti-EGFR antibodies, are for use in the treatment of tumors, *e*.*g*., malignant (neoplastic) tumors. Examples of cancers include but are not limited to, carcinoma, adenoma, blastoma, sarcoma, and lymphoma. More particular examples of such cancers include squamous cell cancer, small-cell lung cancer, non-small cell lung cancer, gastric cancer, pancreatic cancer, glial cell tumors such as glioblastoma and neurofibromatosis, cervical cancer, ovarian cancer, liver cancer, bladder cancer, hepatoma, breast cancer, colon cancer, melanoma, colorectal cancer, endometrial carcinoma, salivary gland carcinoma, kidney cancer, renal cancer, prostate cancer, vulvar cancer, thyroid cancer, hepatic carcinoma and various types of head and neck cancer and other solid and/or metastatic tumors. In a particular embodiment, the invention provides a composition for use in the treatment of a tumor selected from melanoma, breast cancer, ovarian cancer, renal carcinoma, gastrointestinal/colon cancer, lung cancer (*e*.*g*., NSCLC), and prostate cancer and methods for determining the responsiveness of such tumors to EGFR antibody treatments.

Accordingly, theranostic methods predicting which tumors will be responsive to treatment with oligoclonal anti-EGFR antibodies but not responsive to treatment with single monoclonal anti-EGFR antibodies, are provided. Also provided are kits useful for predicting responsiveness of tumors to anti-EGFR monoclonal and oligoclonal antibodies and methods of use thereof.

In one aspect, a monoclonal anti-EGFR antibody preparation is provided comprising a single species of anti-EGFR monoclonal antibody, the preparation being for the treatment of a tumor, a sample obtained from the tumor and tested for RNA levels of EGFR ligands exhibiting levels of mRNAs coding for at least two low affinity EGFR ligands selected from amphiregulin, epigen, or epiregulin in a biopsy sample that are, in average, higher than average levels in the biopsy sample of mRNAs coding for at least two high affinity EGFR ligands selected from betacellulin, EGF, HB-EGF or TGFα. The levels of mRNAs coding for at least two high affinity EGFR ligands may be levels of mRNAs coding for three high affinity ligands. The levels of mRNAs coding for at least two low affinity EGFR ligands may be levels of mRNAs coding for at least three low affinity ligands. The levels of mRNAs coding for at least three low affinity EGFR ligands may be levels of mRNAs coding for four low affinity EGFR ligands. The at the levels of mRNAs coding for least two low affinity EGFR ligands may be levels of mRNAs coding for four low affinity ligands, and the levels of mRNAs coding for at least two high affinity EGFR ligands may be levels of mRNAs coding for three high affinity ligands. In certain embodiments the tumor is malignant. In other embodiments the levels of mRNAs are determined by reverse transcript PCR. The monoclonal anti-EGFR antibody preparation may comprise cetuximab, panitumumab, zalutumumab, matuzumab, or nimotuzumab.

Also provided is an oligoclonal anti-EGFR antibody preparation comprising a plurality of species of monoclonal anti-EGFR antibodies, one against each of at least two extracellular epitopes of EGFR and one of which inhibits binding of EGF to EGFR, the preparation being for treatment of a tumor exhibiting levels of mRNAs coding for at least two high affinity EGFR ligands selected from amphiregulin, epigen, or epiregulin in a biopsy sample that are, in average, higher than average levels in the biopsy sample of mRNAs coding for at least two high affinity EGFR ligands selected from betacellulin, EGF, HB-EGF or TGFα. The oligoclonal anti-EGFR antibody preparation may optionally comprise no more than three different species of monoclonal anti-EGFR antibodies. The oligoclonal anti-EGFR antibody preparation may be a mixture of monoclonal anti-EGFR antibody P1X comprising V_{H} sequence of SEQ ID NO:1 and V_{L} sequence of SEQ ID NO:2, monoclonal anti-EGFR antibody P2X comprising V_{H} sequence of SEQ ID NO:3 and V_{L} sequence of SEQ ID NO:4, and monoclonal anti-EGFR antibody P3X comprising V_{H} sequence of SEQ ID NO:5 and V_{L} sequence of SEQ ID NO:6. In certain embodiments the tumor is a tumor of the skin, central nervous system, head, neck, esophagus, stomach, colon, rectum, anus, liver, pancreas, bile duct, gallbladder, lung, breast, ovary, uterus, cervix, vagina, testis, germ cells, prostate, kidney, ureter, urinary bladder, adrenal, pituitary, thyroid, bone, muscle or connective tissue.

In one aspect, a preparation comprising only a single species of anti-EGFR monoclonal antibody (as opposed to preparations comprising mixtures of monoclonal antibodies) is provided for use in treating a patient having a tumor that is predicted to have a favorable outcome (as described below) as a result of treatment #1, i.e., treatment with the preparation comprising only a single species of monoclonal antibody, and as a result of treatment #2, *i*.*e*., treatment with an oligoclonal anti-EGFR antibody preparation comprising a plurality of species of monoclonal anti-EGFR antibodies, one against each of at least two extracellular epitopes of EGFR, one of which antibody against at least two extracellular epitopes of EGFR being an antibody that inhibits ligand binding to EGFR.

In another aspect, a preparation comprising a plurality of species of monoclonal anti-EGFR antibodies is provided for use in treating a patient having a tumor that is predicted to have a favorable outcome as a result of treatment #2 but not as a result of treatment #1.

Methods for identifying tumors predicted to respond to treatment #1 and treatment #2, or to treatment #2, but not to treatment #1, provided by the invention comprise:
(a) measuring levels in a sample of the tumor (*e*.*g*., a biopsy sample or a circulating tumor cell sample) of at least two low affinity EGFR ligands selected from amphiregulin, epigen, or epiregulin, which may, for each ligand independently, be measured as levels of ligand protein or as levels of the corresponding RNA species coding for the ligand protein, in the biopsy sample;
(b) measuring levels in the sample of at least two high affinity EGFR ligands selected from betacellulin, EGF, HB-EGF or TGFα, which may, for each ligand independently, be measured as protein levels or as levels of the corresponding RNA species coding for them, in the biopsy sample; and
(c) comparing the average level of each of the high affinity EGFR ligands, or of each corresponding RNA species, measured in a) to the average level of each of the low affinity EGFR ligands, or of each corresponding RNA species measured in b) Where, if the average level of low affinity EGFR ligands, or RNAs coding for them, measured in a) is greater than the average level of high affinity EGFR ligands, or RNAs coding for them, measured in b), the low affinity ligands predominate and the patient is predicted to have the favorable outcome as a result of treatment #1 as well as as a result of treatment #2, and if the average level of low affinity EGFR ligands, or RNAs coding for them, measured in a) is less than or equal to the average level of high affinity EGFR ligands, or RNAs coding for them, measured in b), the high affinity ligands predominate and the patient is predicted to have an unfavorable outcome from treatment #1 but is predicted to have a favorable outcome from treatment #2.

In one embodiment, all of the levels measured in a) are protein levels and all of the levels measured in b) are protein levels; in another embodiment all of the levels measured in a) are mRNA levels and all of the levels measured in b) are mRNA levels.

In one embodiment, a monoclonal antibody preparation comprising only a single species of monoclonal antibody is provided for use in the treatment of a patient predicted to have a favorable outcome from treatment with either of the monoclonal anti-EGFR antibody preparation or the oligoclonal anti-EGFR antibody preparation. In another embodiment, an oligoclonal anti-EGFR antibody preparation is provided for use in the treatment of a patient predicted to have a favorable outcome from treatment with either of the monoclonal anti-EGFR antibody preparation or the oligoclonal anti-EGFR antibody preparation.

In yet another embodiment, the oligoclonal anti-EGFR antibody preparation is provided for use in a method for treating a tumor predicted to have an unfavorable outcome from treatment with the monoclonal anti-EGFR antibody preparation.

The monoclonal anti-EGFR antibody preparation comprises a single monoclonal antibody that may be selected from, *e*.*g*., cetuximab, zalutumumab, nimotuzumab, matuzumab and panitumumab, all of which block ligand binding to EGFR, and therefore bind to the same or overlapping EGFR epitopes (Bin 1) and are not suitable for use together in oligoclonal antibody preparations. Members of the plurality of anti-EGFR antibody species in an oligoclonal preparation separately and uniquely bind to two different extracellular epitopes of EGFR, and may separately and uniquely bind to at least three extracellular epitopes of EGFR (in some cases no more than three). Such a plurality may comprise two or three different species of monoclonal anti-EGFR antibodies, and in some embodiments no more than three different species. Mixtures comprising more than one antibody against any one epitope of EGFR are less preferred. In certain embodiments the oligoclonal preparations are duos, trios, or fourfold combinations of antibodies as disclosed in the Oligoclonal Publications. In other embodiments, the oligoclonal preparations provided herein comprise one or more of the anti-EGFR antibodies (*e*.*g*., of the above duos or trios or other oligoclonal combinations) that are not Bin 1 antibodies in combination with one of cetuximab, zalutumumab, nimotuzumab, matuzumab and panitumumab (which are all Bin 1 antibodies).

In another embodiment, if the patient is predicted to have an unfavorable outcome from treatment with the monoclonal anti-EGFR antibody preparation, the patient is subsequently treated with combination therapy comprising separate administration of at least two different monoclonal anti-EGFR antibodies that bind to different extracellular epitopes of EGFR. In certain aspects, the at least two different monoclonal anti-EGFR antibodies are selected from any of the anti-EGFR antibodies disclosed in the Oligoclonal Publications as well as from cetuximab, zalutumumab, nimotuzumab, matuzumab and panitumumab, provided that one of the anti-EGFR antibodies is an antibody that inhibits ligand binding to EGFR.

In another embodiment the tumor is a malignant tumor of the skin, central nervous system, head, neck, esophagus, stomach, colon, rectum, anus, liver, pancreas, bile duct, gallbladder, lung, breast, ovary, uterus, cervix, vagina, testis, germ cells, prostate, kidney, ureter, urinary bladder, adrenal, pituitary, thyroid, bone, muscle or connective tissue.

In another aspect, a monoclonal anti-EGFR antibody preparation comprising a single species of monoclonal antibody is provided, for use in treating a tumor which, prior to administration of the monoclonal antibody, has been determined not to have a predominance of high affinity ligands as compared to the level of low affinity ligands in the tumor.

Also provided are kits for testing a tumor sample to determine levels of both high and low affinity EGFR ligands in the sample. Such a kit may compriseone or more containers comprising; a) at least two pairs of high affinity EGFR ligand-specific polymerase chain reaction (PCR) primers, at least two of the at least two pairs each being specific for a different high affinity EGFR ligand, b) at least two pairs of low affinity EGFR ligand-specific PCR primers, at least two of the at least two pairs each being specific for a different low affinity EGFR ligand, and c) at least one reverse transcription PCR (RT-PCR) reagent. The at least two pairs of high affinity EGFR ligand-specific polymerase chain reaction primers may be specific to at least two of betacellulin, EGF, HB-EGF or TGFα and each of the at least two pairs of low affinity EGFR ligand-specific polymerase chain reaction primers may be specific to at least two of amphiregulin, epigen, or epiregulin. Alternately, the at least two pairs of high affinity EGFR ligand-specific primers consist of primers specific to each of betacellulin, EGF, HB-EGF and TGFα and the at least two pairs of high affinity EGFR ligand-specific primers consist of primers specific to each of amphiregulin, epigen, and epiregulin. The at least one RT-PCR reagent may be one or more of a fluorescent reporter molecule suitable for use in a real-time RT-PCR assay, an RNA-dependent DNA polymerase, a DNA-dependent DNA polymerase, or a solution comprising at least micromolar concentrations of each of deoxyadenosine triphosphate, deoxyguanosine triphosphate, deoxycytidine triphosphate, and deoxythymidine triphosphate. The one or more containers may comprises either or both of at least one container that has an internal temperature of below 20° C and above 0° C, and at least one container that has an internal temperature of below 0° C. The contents of all, or at least one, of the at least one container may have been prepared under cGMP conditions.

In certain aspects of the above embodiments, the oligoclonal anti-EGFR antibody preparation is a composition comprising a trio of anti-EGFR antibodies comprising a first antibody, a second antibody and a third antibody, wherein (i) the first antibody is, or competes for binding to EGFR with, or binds to the same epitope as, an antibody selected from the group consisting of P1X, ca, cb and cc; (ii) the second antibody is, or competes for binding to EGFR with, or binds to the same epitope as, an antibody selected from the group consisting of P2X, cd, ce and cf; and (iii) the third antibody is, or competes for binding to EGFR with, or binds to the same epitope as, an antibody selected from the group consisting of P3X, cg, ch, ci, cj and ck, wherein ca, cb, cd, ce, cf, cg, ch, ci, cj, and ck are each disclosed in PCT Int. Pub. No. WO2011/140254. The amino acid sequences of the antibodies, in particular the sequences of the six CDR regions and the heavy and light chain variable region sequences of these antibodies, disclosed in W02011/140254 are incorporated herein by reference. Antibodies that bind to the same epitope as the indicated antibodies include antibodies that comprise the same CDR sequences as these antibodies and affinity matured variants thereof.

Other features and advantages of the invention will be apparent from the following detailed description, and from the claims.

### Brief Description of the Figures

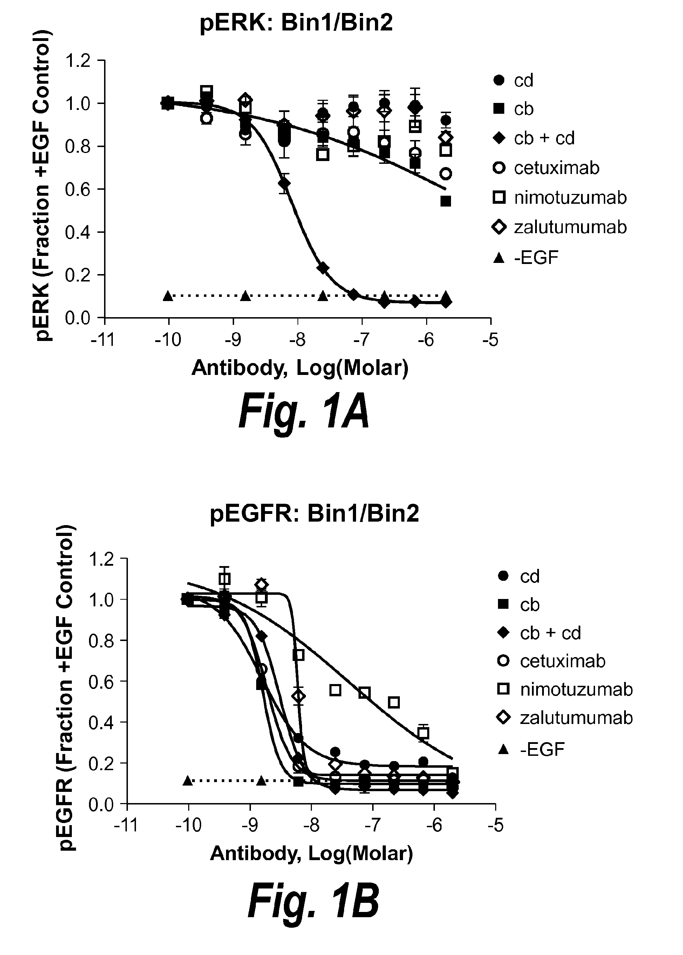

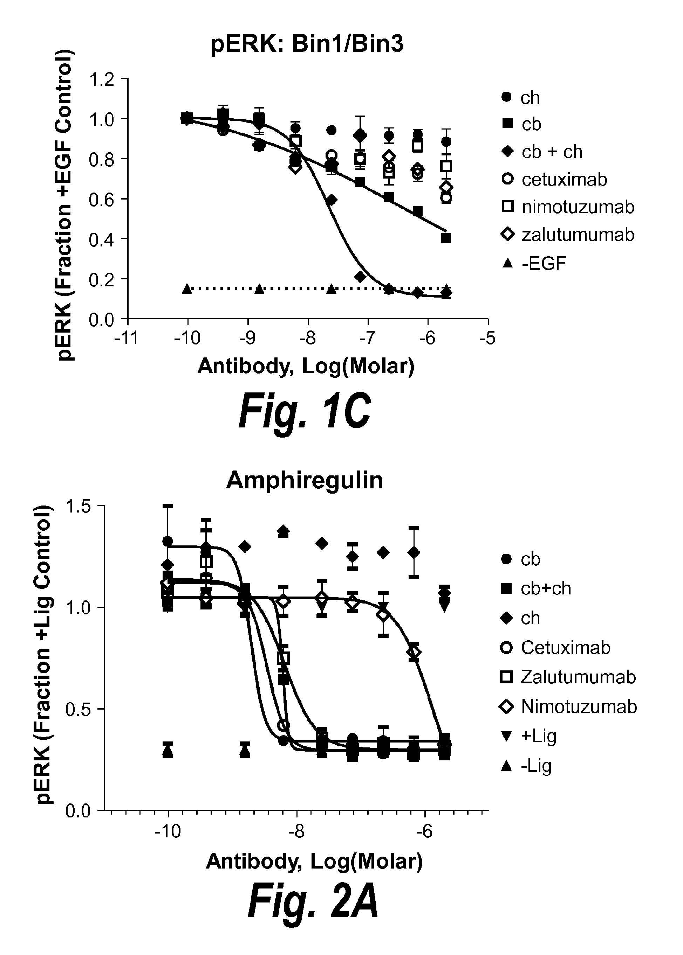

Figures 1A - 1C: Phospho-EGF receptor and phospho-ERK signaling inhibition by single and pairwise combinations of Bin 1 + Bin 2 or Bin 1 + Bin 3 antibodies and comparisons with other known anti-EGFR antibodies such as cetuximab, nimotuzumab, and zalutumumab. Figure 1A shows inhibition of ERK activation by the Bin1/2 antibodies cb and cd. Figure 1B shows the inhibition of EGFR activation by the Bin1/2 antibodies and cd. Figure 1C shows the inhibition of ERK activation by the Bin1/3 antibodies cb and ch. Lines depict a five parameter logistic fit to the data from each combination.

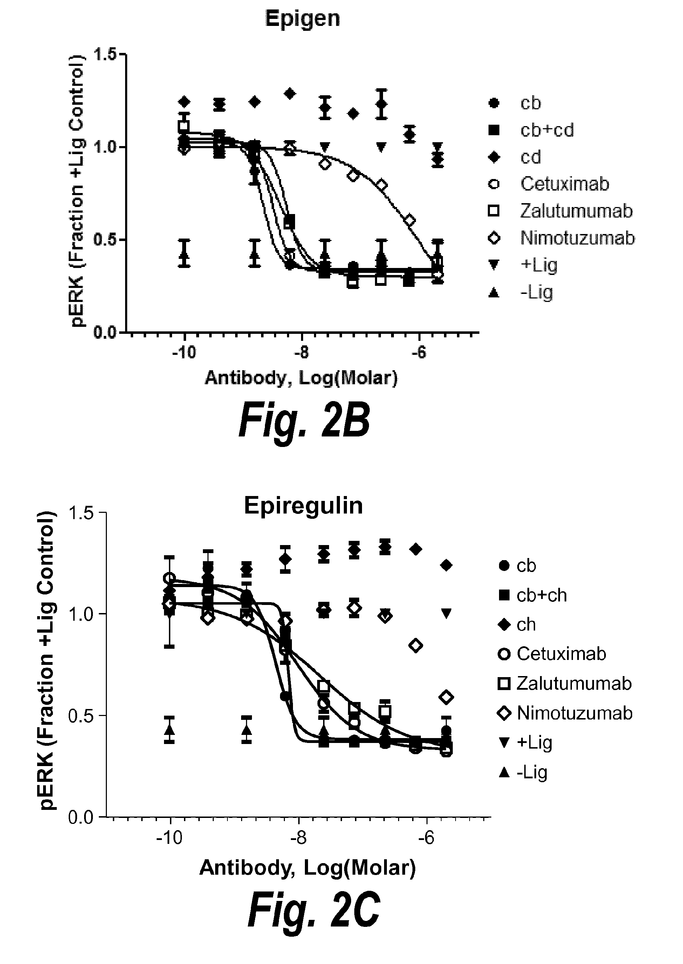

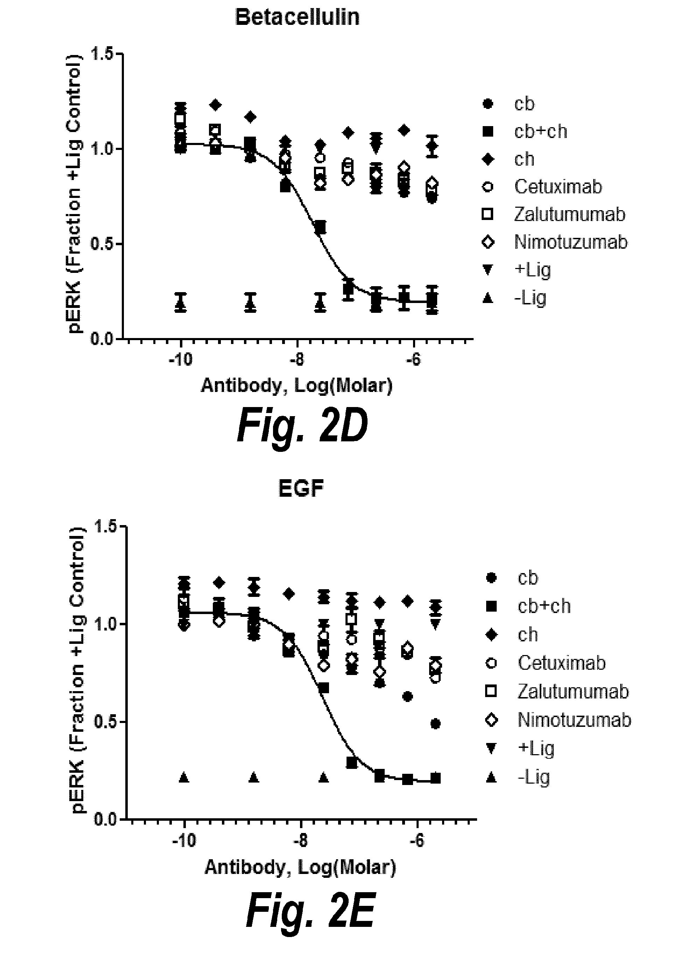

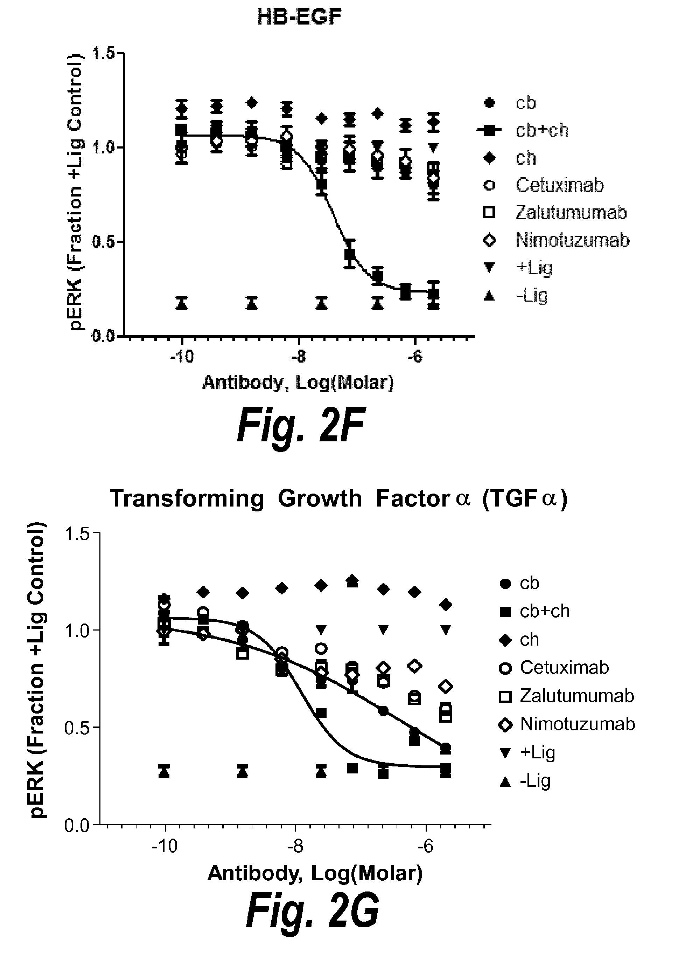

Figures 2A - 2G: Inhibition of ligand-mediated tumor cell signaling in A431 cells preincubated with varying concentrations of anti-EGFR monoclonal antibodies cb (Bin1), cd (Bin2), cetuximab, zalutumumab, or nimotuzumab; as well as the oligoclonal combination of cb + cd; for 2 hrs. After incubation cells were stimulated with an EGFR ligand (8 nanomolar final concentration) for about 10 minutes. Figures show ELISA analysis of phospho-ERK (pERK) production (y-axis) as a function of antibody concentration (x-axis, in Log Molar concentration) after stimulation with the ligands amphiregulin (Figure 2A), epigen (Figure 2B), epiregulin (Figure 2C), betacellulin (Figure 2D), epidermal growth factor (EGF, Figure 2E), heparin-binding EGF-like growth factor (HB-EGF, Figure 2F), or transforming growth factor α (TGF-α, Figure 2G). A431 cells incubated in the absence of anti-EGFR antibodies but with the ligand indicated in each graph (+Lig) or without ligand stimulation (-Lig) were used as positive and negative controls, respectively.

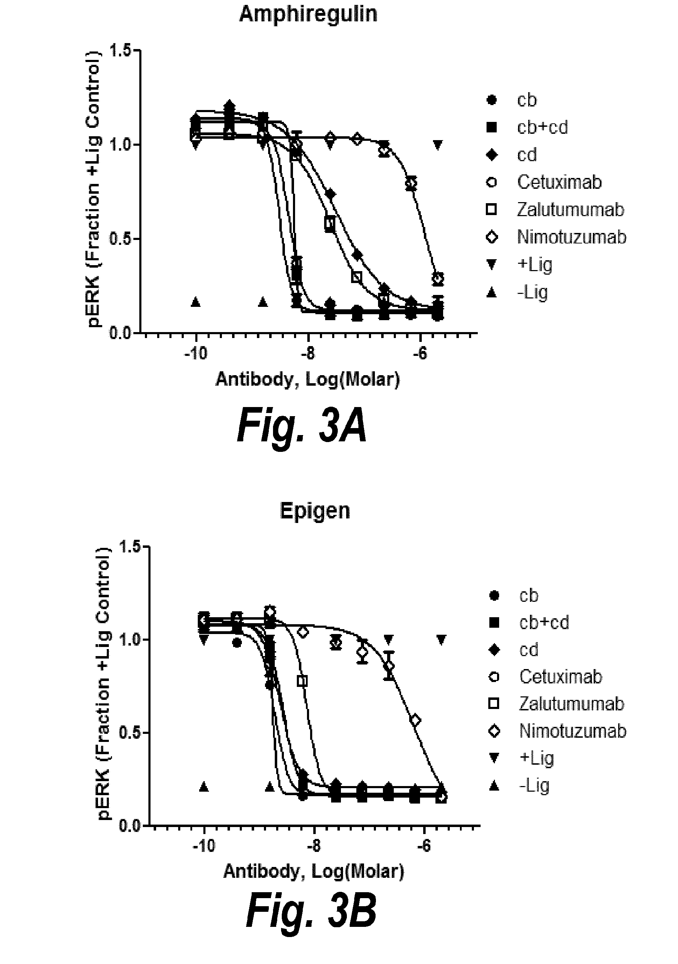

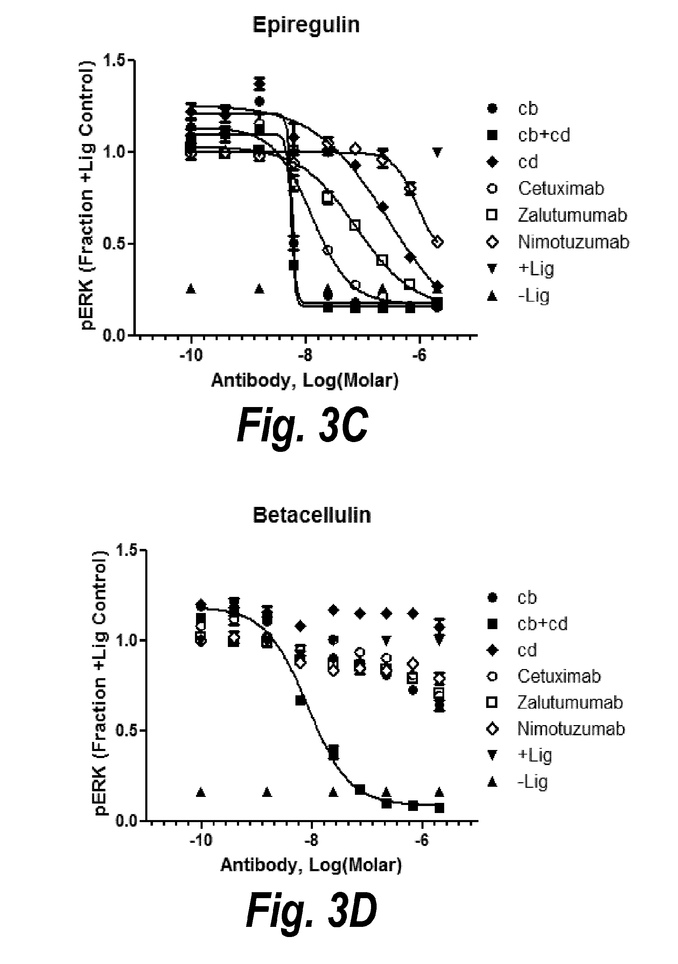

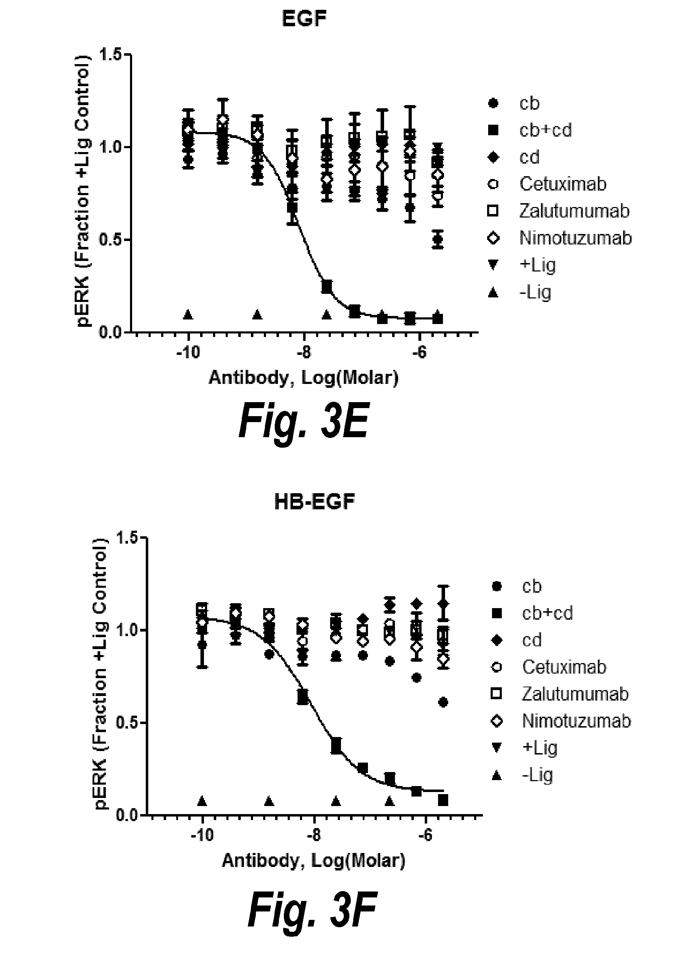

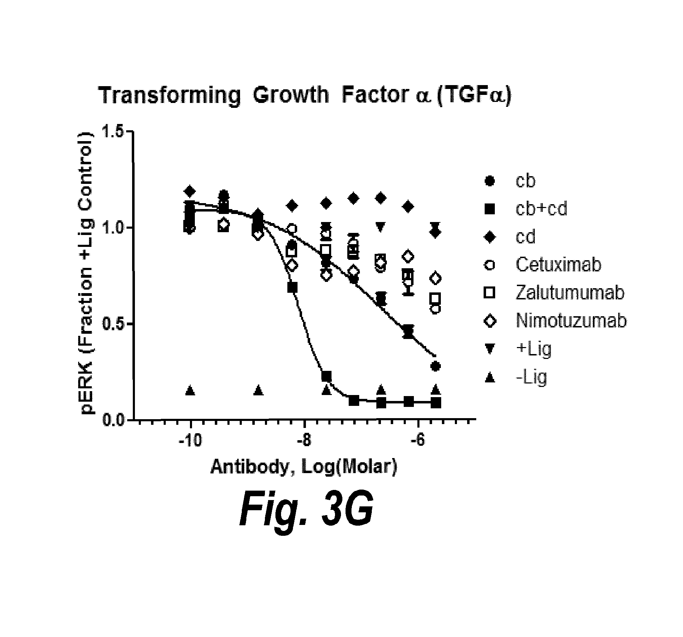

Figures 3A - 3G: Inhibition of ligand-mediated tumor cell signaling in A431 cells preincubated with varying concentrations of anti-EGFR monoclonal antibodies cb (Bin1), ch (Bin3), cetuximab, zalutumumab, or nimotuzumab; as well as the oligoclonal combination of cb + ch; for 2 hrs. After pre-incubation with antibodies, cells were stimulated with an EGFR ligand (8 nanomolar final concentration) for 10 minutes. Figures show ELISA analysis ofphospho-ERK (pERK) production (y-axis) as a function of antibody concentration (x-axis, in Log Molar concentration) after stimulation with the ligands amphiregulin (Figure 3A), epigen (Figure 3B), epiregulin (Figure 3C), betacellulin (Figure 3D), epidermal growth factor (EGF, Figure 3E), heparin-binding EGF-like growth factor (HB-EGF, Figure 3F), or transforming growth factor α (TGF-α, Figure 3G). A431 cells incubated in the absence of anti-EGFR antibodies but with the ligand indicated in each graph (+Lig) or without ligand stimulation (-Lig) were used as positive and negative controls, respectively.

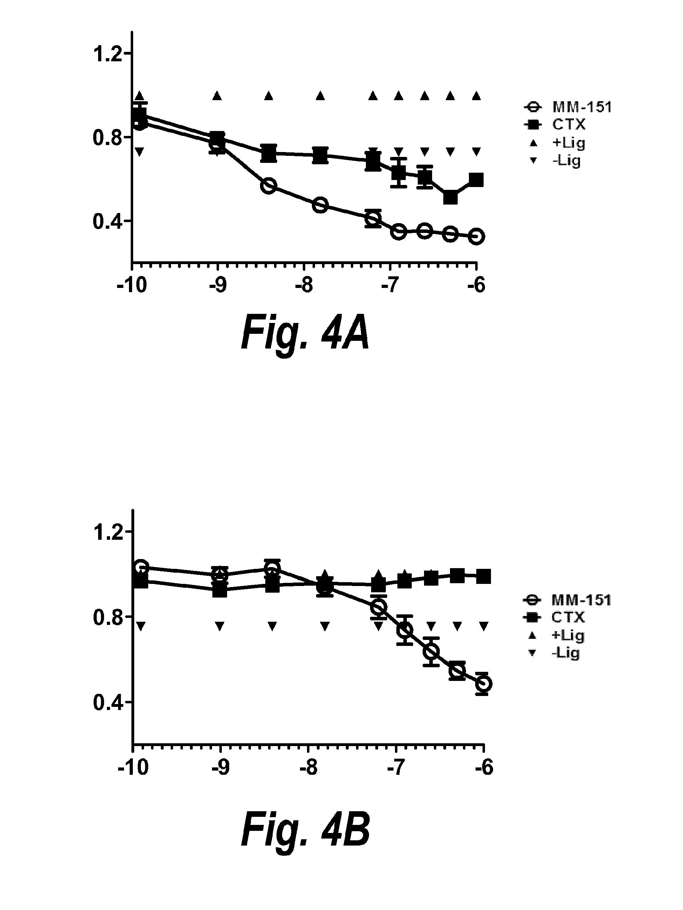

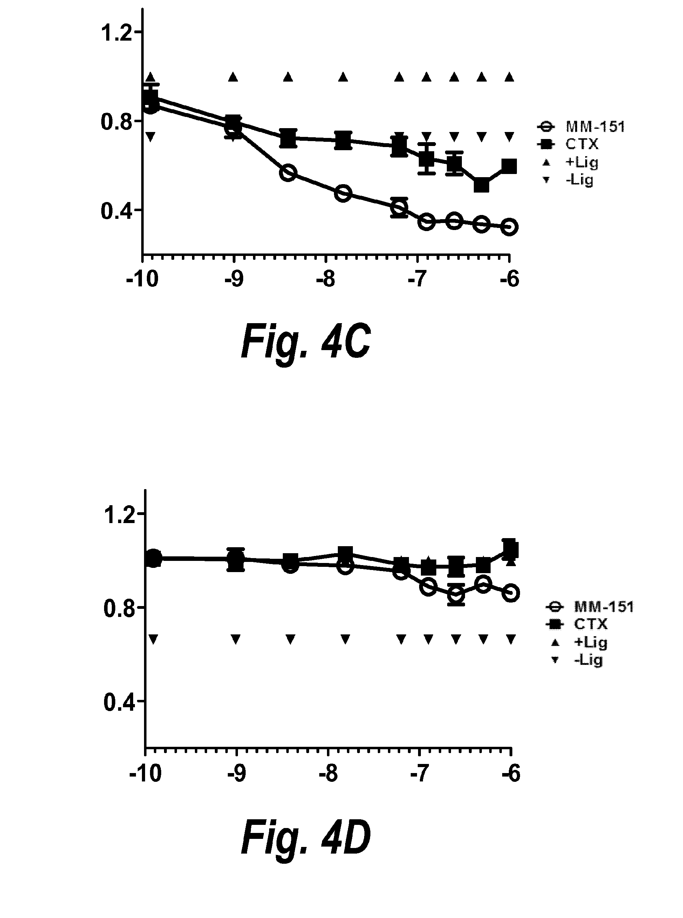

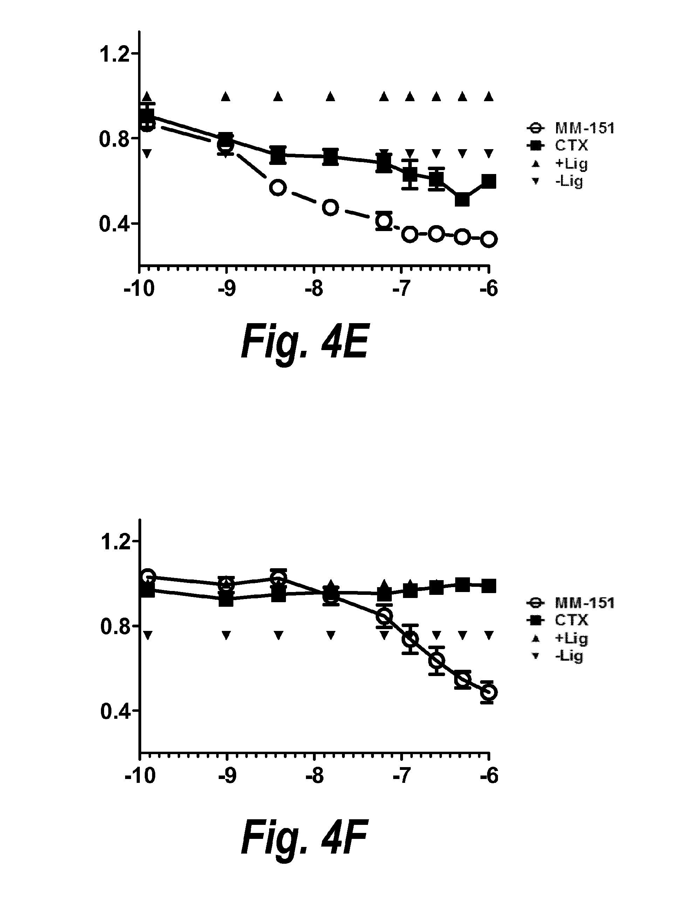

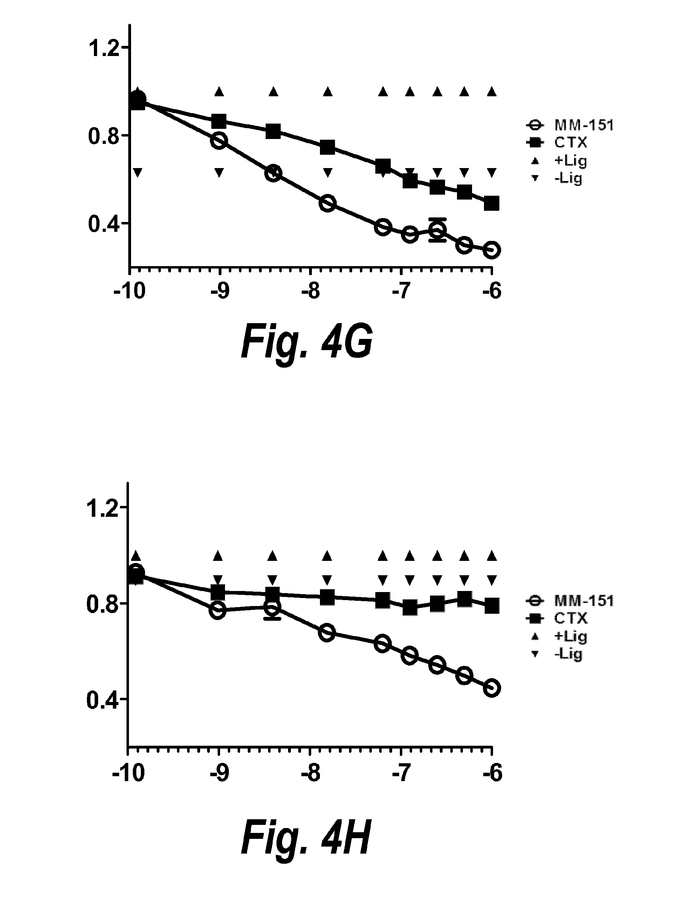

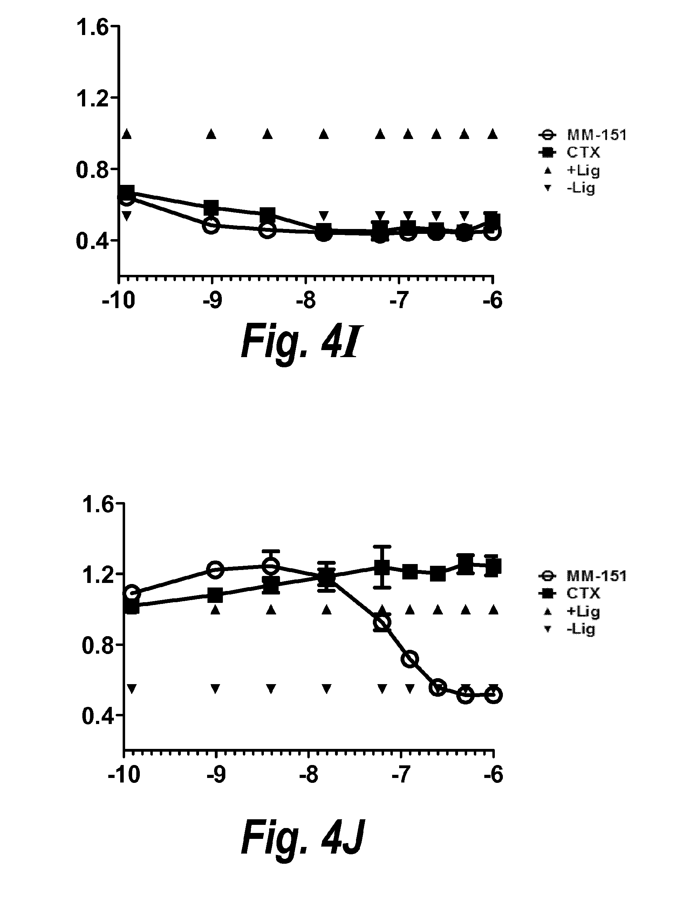

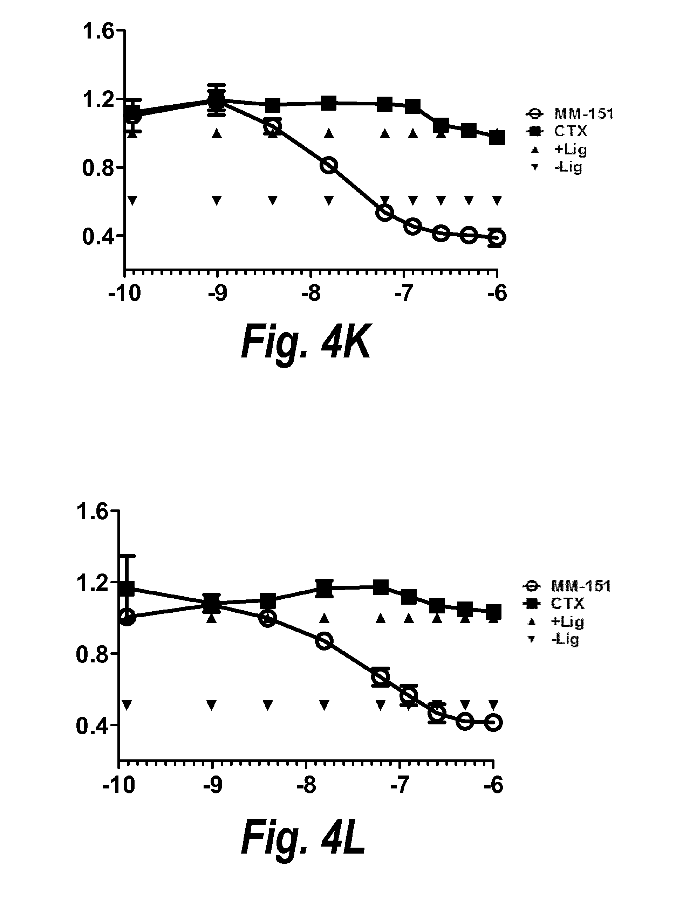

Figures 4A ― 4L: Inhibition of high affinity EGFR ligand-mediated tumor cell proliferation. H322M cells (Figures 4A-4D), H1975 cells (Figures 4E-4H), and LIM1215 cells (Figures 4I-4L) were treated with varying concentrations of anti-EGFR monoclonal and oligoclonal antibodies in the presence of EGFR ligands. Cells were treated with 200ng/ml amphiregulin (AREG) (Figures 4A, 4E, and 4I), 50ng/ml EGF (Figures 4B, 4F, and 4J), 50ng/ml TGFα (Figures 4C, 4G, and 4K) or 90ng/ml HB-EGF (Figures 4D, 4H, and 4L) in the presence of varying concentrations of MM-151 (open circles or cetuximab (CTX, solid squares; Bristol-Myers Squibb). Cells treated with ligand (+Lig, upward arrow) or without ligand (-Lig, downward arrow) in the absence of antibody treatment served as controls. The y-axes represent cell viability as the fraction of the viability of the amphiregulin -treated control cells and the x-axes represent antibody concentration in Log (Molar).

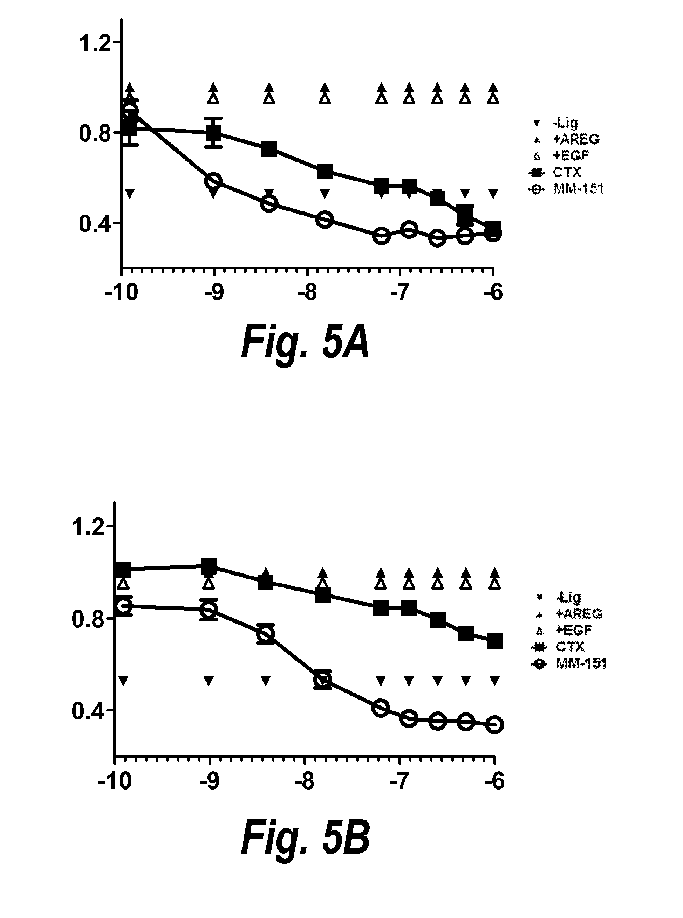

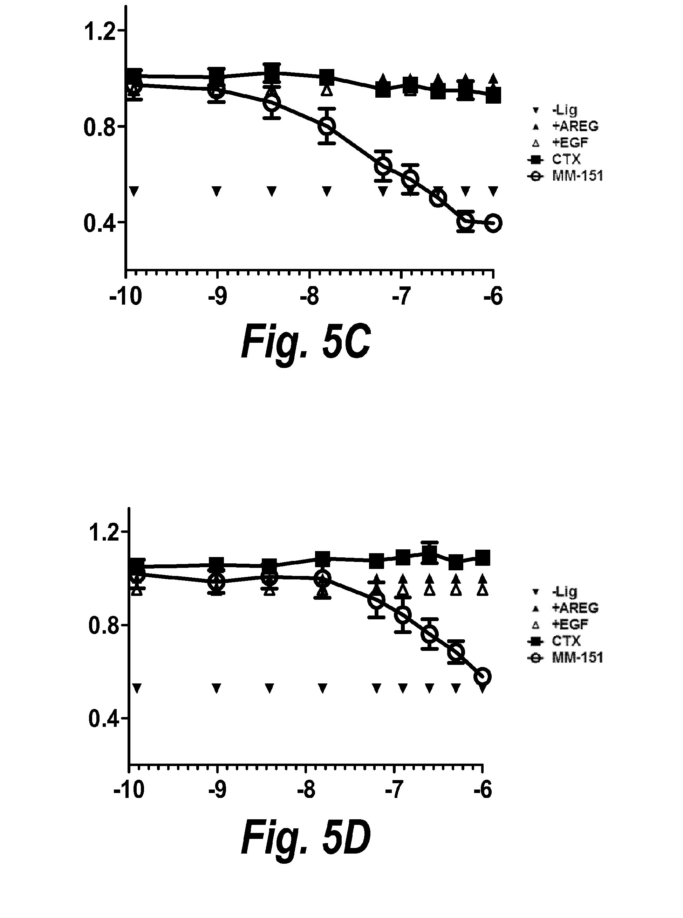

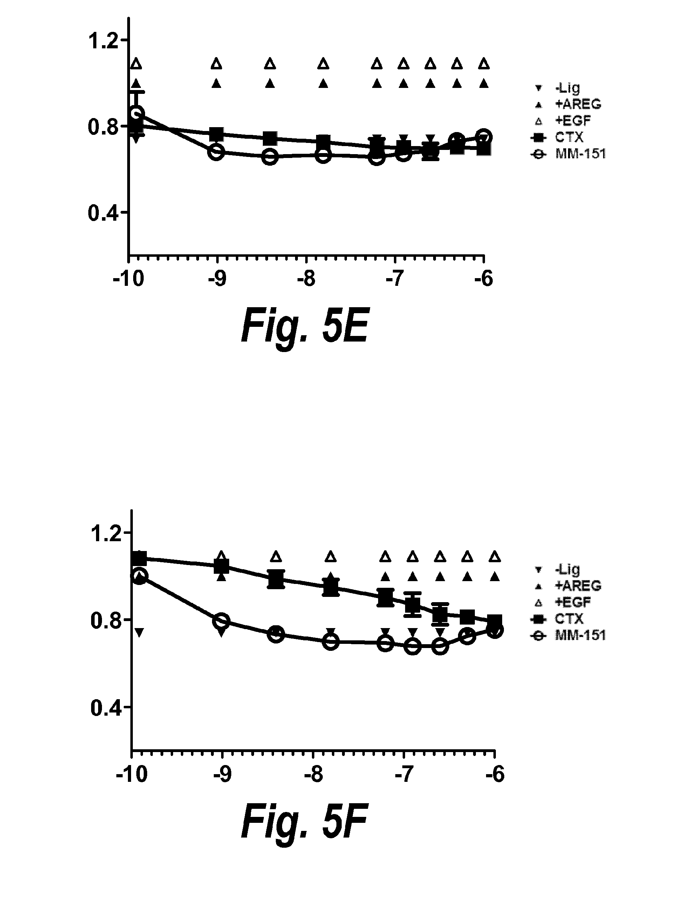

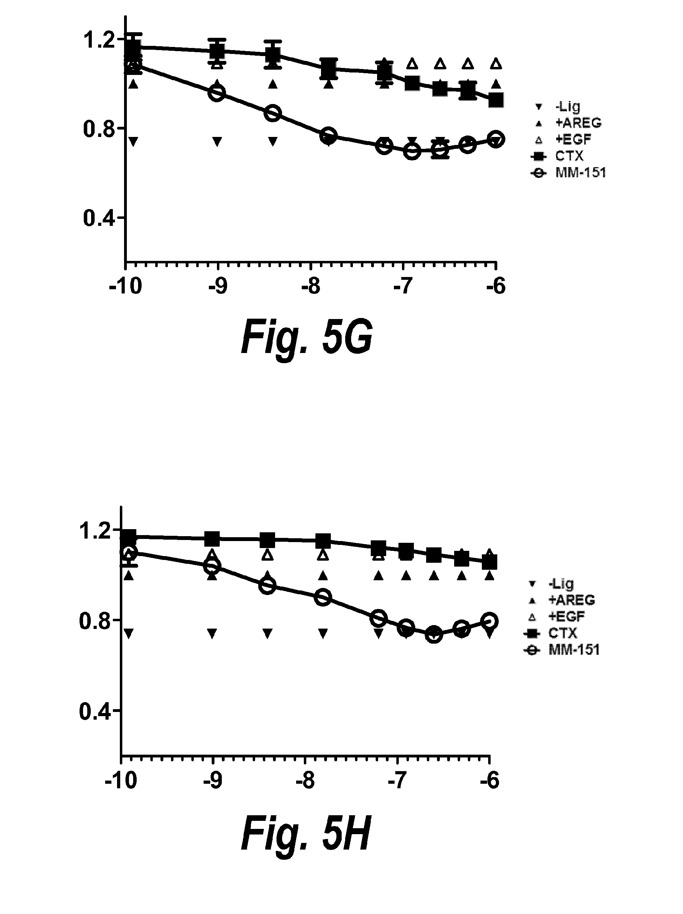

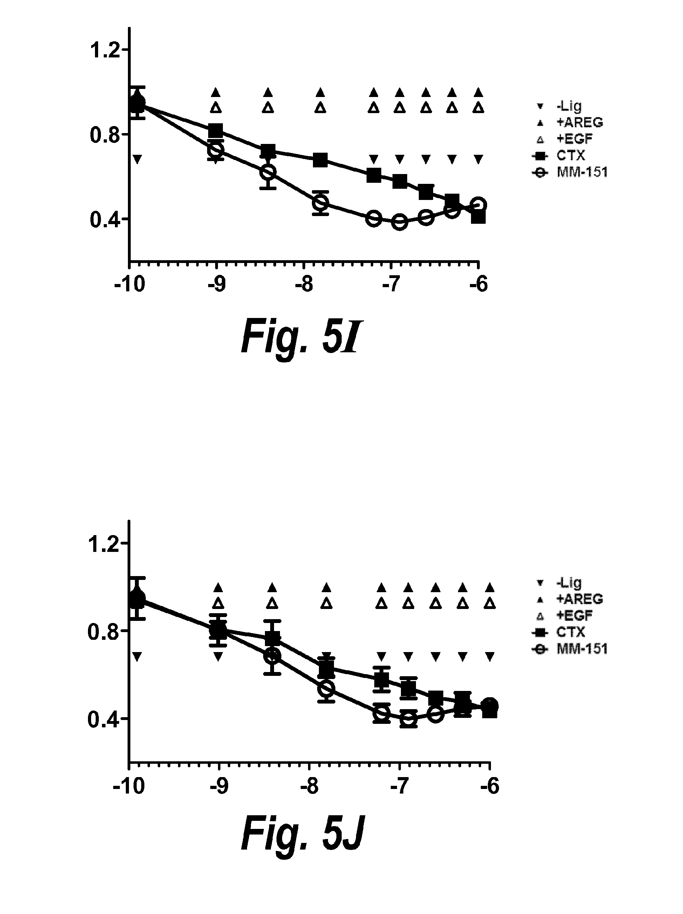

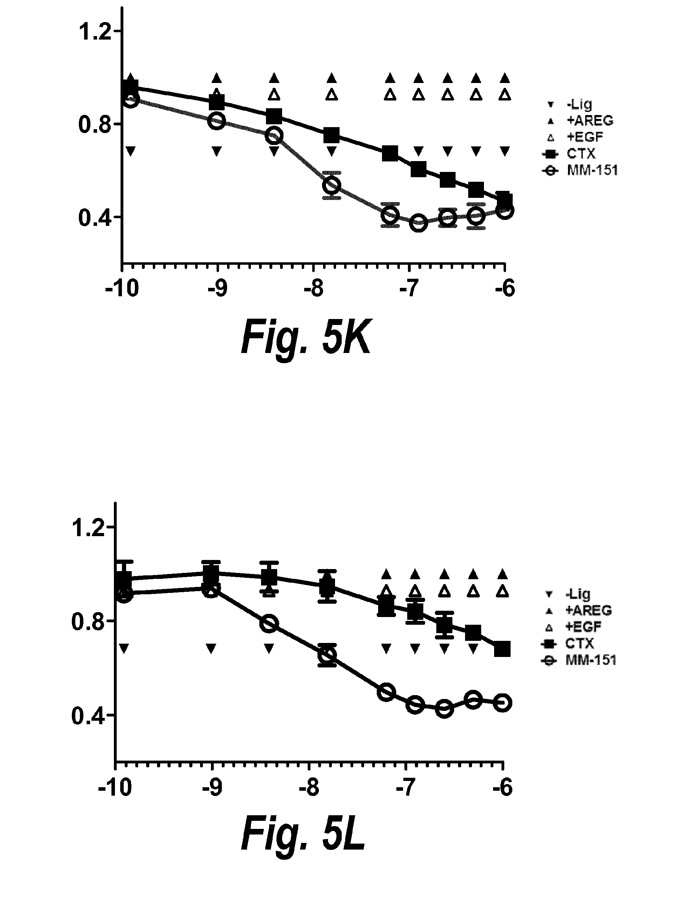

Figures 5A ― 5L: Effect of EGFR high affinity ligand titration on cell responsiveness to anti-EGFR inhibitors *in vitro.* The non-small cell lung cancer (NSCLC) lines H322M (Figures 5A - 5D), HCC827 (Figures 5E-5H), and H1975 (Figures 5I-5L) were tested. Controls were growth in media with amphiregulin alone (+AREG, 200ng/ml) or EGF alone as a control (+EGF, 20ng/ml) or no added ligand (-Lig). Treatments were with varying concentrations (0.1-1 µM final concentration) of MM-151 or cetuximab (CTX) in the following conditions: amphiregulin alone (200ng/ml, Figures 5A, 5E, and 5I); a 1000:1 amphiregulin:EGF ratio (0.2ng/ml EGF, Figures 5B, 5F, and 5J); a 100:1 amphiregulin:EGF ratio (2ng/ml EGF, Figures 5C, 5G, and 5K); and a 10:1 amphiregulin:EGF ratio (20ng/ml EGF, Figures 5D, 5H, and 5L). The y-axes represent cell viability as a fraction of the viability of the AREG-treated control cells, whereas and the x-axes represent antibody concentration in Log (Molar).

### Detailed Description

### I. Definitions

The terms "EGFR," and "EGF receptor" are used interchangeably herein to refer to human EGFR protein (also referred to as ErbB1 or HER1); see UniProtKB/Swiss-Prot entry P00533.

The term "monoclonal antibody" as used herein refers to an antibody obtained from a population of substantially homogeneous antibodies, *i*.*e*., the individual antibodies comprising the population are identical except for possible naturally occurring mutations that may be present in minor amounts. Such antibodies may be obtained, e.g. from hybridomas or by recombinant expression. Antigen binding fragments (including scFvs) of such immunoglobulins are also encompassed by the term "monoclonal antibody" as used herein. Monoclonal antibodies are highly specific, generally being directed against a single epitope on a single antigen site, *e*.*g*., on the extracellular domain of EGFR. Monoclonal antibodies include chimeric antibodies - whose variable regions derive from a first animal species (*e*.*g*., mouse) and whose constant regions derive from a second animal species (*e*.*g*., human), human antibodies and humanized antibodies.

The terms "treat," "treating," and "treatment," as used herein, refer to therapeutic or preventative measures described herein. The methods of "treatment" employ administration to a subject, an antibody or antibody pair or trio disclosed herein, for example, a subject having a disorder associated with EGFR dependent signaling or predisposed to having such a disease or disorder, in order to prevent, cure, delay, reduce the severity of, or ameliorate one or more symptoms of the disease or disorder or recurring disease or disorder, or in order to prolong the survival of a subject beyond that expected in the absence of such treatment.

Commercially available pharmaceutical anti-EGFR antibodies include cetuximab, panitumumab and nimotuzumab (which is not yet available in the US market). Other pharmaceutical anti-EGFR antibodies include zalutumumab, and matuzumab, which are in development. Still other anti-EGFR antibodies include those disclosed in the Oligoclonal Publications, e.g., the antibodies disclosed below.

P1X is a human IgG1 having a heavy chain variable region comprising SEQ ID NO: 1 and a light chain variable region comprising SEQ ID NO: 2;

P2X is a human IgG1 having a heavy chain variable region comprising SEQ ID NO: 3 and a light chain variable region comprising SEQ ID NO: 4; and

P3X is a human IgG1 having a heavy chain variable region comprising SEQ ID NO: 5 and a light chain variable region comprising SEQ ID NO: 6.

"MM-151" indicates a triple combination of P1X + P2X + P3X at a P1X:P2X:P3X molar ratio of 2:2:1.

The term "patient" includes human and other mammalian subjects that receive either prophylactic or therapeutic treatment.

The term "sample" refers to tissue, body fluid, or a cell (or a fraction of any of the foregoing) taken from a patient. Normally, the tissue or cell will be removed from the patient, but *in vivo* diagnosis is also contemplated. In the case of a solid tumor, a tissue sample can be taken from a surgically removed tumor and prepared for testing by conventional techniques. In the case of lymphomas and leukemias, lymphocytes, leukemic cells, or lymph tissues can be obtained (*e*.*g*., leukemic cells from blood) and appropriately prepared. Other samples, including urine, tears, serum, plasma, cerebrospinal fluid, feces, sputum, cell extracts *etc*. can also be useful for particular cancers.

Various aspects of the disclosure are described in further detail in the following subsections.

### II. Outcomes

A patient having a tumor predicted by the methods disclosed herein to have a favorable outcome following treatment with a monoclonal or oligoclonal anti-EGFR antibody, and who is then treated accordingly, may exhibit one of the following responses to therapy:
- Pathologic complete response (pCR): absence of invasive cancer following primary systemic treatment.
- Complete Response (CR): Disappearance of all target lesions.
- Partial Response (PR): At least a 30% decrease in the sum of dimensions of target lesions, taking as reference the baseline sum diameters; or
- Stable Disease (SD): Neither sufficient shrinkage to qualify for partial response, nor sufficient increase to qualify for progressive disease, taking as reference the smallest sum diameters while on study.

In exemplary outcomes, patients treated as disclosed herein may experience improvement in at least one sign of cancer.

In one embodiment the patient so treated exhibits pCR, CR, PR, or SD.

In another embodiment, the patient so treated experiences tumor shrinkage and/or decrease in growth rate, *i*.*e*., suppression of tumor growth. In another embodiment, unwanted cell proliferation is reduced or inhibited. In yet another embodiment, one or more of the following can occur: the number of cancer cells can be reduced; tumor size can be reduced; cancer cell infiltration into peripheral organs can be inhibited, retarded, slowed, or stopped; tumor metastasis can be slowed or inhibited; tumor growth can be inhibited; recurrence of tumor can be prevented or delayed; one or more of the symptoms associated with cancer can be relieved to some extent.

In other embodiments, such improvement is measured by a reduction in the quantity and/or size of measurable tumor lesions. Measurable lesions are defined as those that can be accurately measured in at least one dimension (longest diameter is to be recorded) as ≥10 mm by CT or MRI scan (*e*.*g*., CT scan slice thickness no greater than 5 mm), 10 mm caliper measurement by clinical exam or >20 mm by chest X-ray. The size of non-target lesions can also be measured for improvement. In one embodiment, lesions can be measured on x-rays or CT or MRI images.

In other embodiments, cytology or histology can be used to evaluate responsiveness to a therapy. The cytological confirmation of the neoplastic origin of any effusion that appears or worsens during treatment when the measurable tumor has met criteria for response or stable disease can be considered to differentiate between response or stable disease (an effusion may be a side effect of the treatment) and progressive disease.

In some embodiments, a beneficial response to therapy is indicated by at least one therapeutic effect selected from the group consisting of reduction in size of a tumor, reduction in number of metastatic lesions appearing over time, complete remission, partial remission, stable disease, increase in overall response rate, or a pathologic complete response.

### III. Pharmaceutical Compositions

Pharmaceutical compositions for use in the methods provided for herein are commercially available anti-EGFR compositions, e.g., of cetuximab, panitumumab and nimotuzumab, as well as the various pharmaceutical compositions provided in the Oligoclonal Publications.

In another aspect, herein provided is a pharmaceutical composition (*e*.*g*., formulated with a pharmaceutically acceptable carrier) containing only a single monoclonal antibody against EGFR, said composition being for the treatment of a tumor comprising a preponderance of low affinity EGFR ligands as compared to levels of high affinity EGFR ligands. Also provided is a pharmaceutical composition containing a combination of at least two anti-EGFR monoclonal antibodies that each binds to a different extracellular epitope of EGFR.

As used herein, "pharmaceutically acceptable carrier" includes any and all solvents, dispersion media, coatings, antibacterial and antifungal agents, isotonic and absorption delaying agents, excipients and the like that are physiologically compatible. Preferably, the carrier is suitable for intravenous, intramuscular, subcutaneous, parenteral, spinal or epidermal administration (*e*.*g*., by injection or infusion).

Compositions can be administered alone or in combination therapy, *i*.*e*., combined with other therapeutic agents. For example, the combination therapy can include a composition provided herein with at least one or more additional therapeutic agents, such as the anti-cancer agents. The compositions can also be administered in conjunction with (*e*.*g*., either before or after) radiation therapy and/or surgery. Particular combinations of anti-EGFR antibodies may also be administered separately or sequentially, with or without additional therapeutic agents.

Compositions can be administered by a variety of methods known in the art, with the route and/or mode of administration varying depending upon the desired results.

### IV. Antibodies

Provided herein are monoclonal anti-EGFR antibody preparations comprising only a single species of anti-EGFR antibody for use in treating a tumor comprising a preponderance of low affinity EGFR ligands as compared to levels of high affinity EGFR ligands. Use of oligoclonal anti-EGFR antibodies for the treatment of a disease associated with high-affinity EGFR ligand-driven signaling is also provided, as is use of oligoclonal anti-EGFR antibodies for the treatment of tumors comprising protein or mRNA levels of at least two high-affinity EGFR ligands that are higher than levels in the tumor of at least two low-affinity EGFR ligands.

The monoclonal or oligoclonal antibody can be administered alone or with another therapeutic agent that acts in conjunction with or synergistically with the oligoclonal antibody to treat the disease associated with EGFR-mediated signaling.

Exemplary Antibodies that may be administered as a single species include the known antibodies cetuximab, panitumumab, zalutumumab, matuzumab and nimotuzumab. The sequences of the heavy and light chain variable regions of zalutumumab and nimotuzumab are shown in the Table below. The Table below also shows the heavy and light chain variable region sequences of three exemplary antibodies that may be included in the oligoclonal antibody preparations for use according to the invention. P1X, P2X and P3X are affinity matured variants of antibodies described in W02011/140254 such as those used in the Examples.

| | | |
|---|---|---|
| P1X V_{H} | MGFGLSWLFLVAILKGVQC | SEQ ID NO: 1 |
| | QVQLVQSGAEVKKPGSSVKV | |
| | SCKASGGTFSSYAISWVRQA | |
| | PGQGLEWMGSIIPIFGTVNY | |
| | AQKFQGRVTITADESTSTAY | |
| | MELSSLRSEDTAVYYCARDP | |
| | SVNLYWYFDLWGRGTLVTVSS | |
| P1X V_{L} | MGTPAQLLFLLLLWLPDTTG | SEQ ID NO: 2 |
| | DIQMTQSPST LSASVGDRVT | |
| | ITCRASQSISSWWAWYQQKP | |
| | GKAPKLLIYDASSLESGVPS | |
| | RFSGSGSGTEFTLTISSLQP | |
| | DDFATYYCQQYHAHPTTFGG | |
| | GTKVEIK | |
| P2X V_{H} | MGFGLSWLFLVAILKGVQC | SEQ ID NO: 3 |
| | QVQLVQSGAEVKKPGSSVKV | |
| | SCKASGGTFGSYAISWVRQA | |
| | PGQGLEWMGSIIPIFGAANP | |
| | AQKSQGRVTITADESTSTAY | |
| | MELSSLRSEDTAVYYCAKMG | |
| | RGKVAFDIWGQGTMVTVSS | |
| P2X V_{L} | MGTPAQLLFLLLLWLPDTTG | SEQ ID NO: 4 |
| | DIVMTQSPDSLAVSLGERAT | |
| | INCKSSQSVLYSPNNKNYLA | |
| | WYQQKPGQPPKLLIYWASTR | |
| | ESGVPDRFSGSGSGTDFTLT | |
| | ISSLQAEDVAVYYCQQYYGS | |
| | PITFGGGTKVEIK | |
| P3X V_{H} | MGFGLSWLFLVAILKGVQC | SEQ ID NO: 5 |
| | QVQLVQSGAEVKKPGASVKV | |
| | SCKASGYAFTSYGINWVRQA | |
| | PGQGLEWMGWISAYNGNTYY | |
| | AQKLRGRVTMTTDTSTSTAY | |
| | MELRSLRSDDTAVYYCARDL | |
| | GGYGSGSVPFDPWGQGTLVTVSS | |
| P3X V_{L} | MGTPAQLLFLLLLWLPDTTG | SEQ ID NO: 6 |
| | EIVMTQSPATLSVSPGERAT | |
| | LSCRASQSVSSNLAWYQQKP | |
| | GQAPRLLIYGASTRATGIPA | |
| | RFSGSGSGTEFTLTISSLQS | |
| | EDFAVYYCQDYRTWPRRVFG | |
| | GGTKVEIK | |
| zalutumumab V_{H} | QVQLVESGGGVVQPGRSLRLSCAASG | SEQ ID NO: 7 |
| | FTFSTYGMHWVRQAPGKGLEWVAVIW | |
| | DDGSYKYYGDSVKGRFTISRDNSKNTL | |
| | YLQMNSLRAEDTAVYYCARDGITMVR | |
| | GVMKDYFDYWGQGTLVTVSS | |
| zalutumumab V_{L} | AIQLTQSPSSLSASVGDRVTITCRASQDISSAL | SEQ ID NO: 8 |
| | VWYQQKPGKAPKLLIYDASSLESGVPSRFSG | |
| | SESGTDFTLTISSLQPEDFATYYCQ | |
| | QFNSYPLTFGGGTKVEIK | |
| Nimotuzumab V_{H} | QVQLQQPGAELVKPGASVKLSCKASGYTFTNY | SEQ ID NO: 9 |
| | YIYWVKQRPGQGLEWIGGINPTSGGSNFNEKFK | |
| | TKATLTVDESSTTAYMQLSSLTSEDSAVYYCTR | |
| | QGLWFDSDGRGFDFWGQGTTLTVSS | |
| Nimotuzumab V_{L} | DVLMTQIPLSLPVSLGDQASISCRSSQNI | SEQ ID NO: 10 |
| | VHSNGNTYLDWYLQKPGQSPNLLIYKV | |
| | SNRESGVPDRFRGSGSGTDFTLKISRVEA | |
| | EDLGVYYCFQYSHVPWTFGGGTKLEIK | |

Also provided are kits for testing a tumor sample, *e*.*g*., a tumor biopsy sample or a circulating tumor cell, to determine levels of both high and low affinity EGFR ligands in the sample, said kits being comprised by one or more containers comprising;
a) at least two pairs of high affinity EGFR ligand-specific polymerase chain reaction (PCR) primers,
b) at least two pairs of low affinity EGFR ligand-specific PCR primers, and
c) at least one reverse transcription PCR (RT-PCR) reagent.

In another embodiment, the kit may further contain instructions for use in determining how to treat a tumor in a patient following determination of levels of high and low affinity ligands in a sample of the tumor. The kit may include an indication of the intended use of the contents of the kit (e.g., in the form of a label or other printed or recorded matter).

Other embodiments are described in the following non-limiting Examples.

### Examples

### Materials and Methods

Throughout the examples, the following materials and methods are used unless otherwise stated.

In general, unless otherwise indicated, conventional techniques of chemistry, molecular biology, recombinant DNA technology, immunology (especially, *e*.*g*., antibody technology), and standard techniques in polypeptide preparation are used. See, *e*.*g*., Sambrook, Fritsch and Maniatis, Molecular Cloning Cold Spring Harbor Laboratory Press (1989); Antibody Engineering Protocols (Methods in Molecular Biology), 510, Paul, S., Humana Pr (1996); Antibody Engineering: A Practical Approach (Practical Approach Series, 169), McCafferty, Ed., Irl Pr (1996); Antibodies: A Laboratory Manual, Harlow et al., C.S.H.L. Press, Pub. (1999); and Current Protocols in Molecular Biology, eds. Ausubel et al., John Wiley & Sons (1992).

### Pulverization of Tumor Cells

A cryopulverizer (COVARIS Inc.) is used for the pulverization of tumors. Tumors are stored in special bags (pre-weighed before the addition of the tumor) and placed in liquid nitrogen while handling them. For small tumors, 200 µL of Lysis buffer is first added to the bag containing the tumor, frozen in liquid nitrogen and then pulverized to improve the recovery of the tumor from the bag. Pulverized tumors are transferred to 2 mL EPPENDORF tubes and placed in liquid nitrogen until ready for further processing.

### Lysis of Tumor Cells

Tumors are lysed in Lysis buffer supplemented with protease and phosphatase inhibitors. Lysis Buffer is added to the tumor aliquots in a final concentration of about 62.5 mg/mL. Tumor samples are homogenized by vortexing for 30 sec and incubating on ice for about 30 min. The lysates are spun for about 10 min in Qiagen QIASHREDDER columns for further homogenization of the samples. Cleared lysates are aliquoted into fresh tubes for further processing.

### Measurement of Inhibition of EGFR Ligand-Mediated phosphorylation of ERK in Tumor Cells

Inhibition of ligand-mediated tumor cell signaling is investigated as follows: A431 (ATCC CRL-1555™) epidermoid carcinoma cells are seeded at a density of 35,000 cells/well or 17,500 cells per half well in 96 well tissue culture plates and grown in DMEM medium supplemented with antibiotics, 2 mM L-glutamine and 10% fetal bovine serum (FBS) for 24 hours at 37°C and 5% carbon dioxide. Cells are serum starved in 1% FBS medium with antibiotics and 2mM L-glutamine for about 20 hours at 37°C and 5% carbon dioxide. Cells are then treated as described below in each Example. Cells are washed with ice-cold PBS and lysed in 50µl ice-cold Lysis buffer (Mammalian Protein Extraction Lysis Reagent (M-PER, Pierce, Thermo Scientific product # 78505) amended with 150mM NaCl and protease inhibitor cocktail (Sigma, P714)) by incubating on ice for 30 minutes. Lysates are either analyzed immediately by ELISA for phospho-ERK (a downstream effector of EGFR) or frozen at -80°C until use.

### ELISA Assays

For the phospho-EGFR sandwich ELISA, 96-half well GREINER high binding plates (Cat. #675077; GREINER BIO-ONE, Monroe, NC) are coated with 50 µL of an EGFR antibody (4µg/ml final concentration; EGFR Ab-11, Clone: 199.12, without BSA and azide, Fisher Scientific, cat# MS396P1ABX), and incubated overnight at room temperature. Next morning, plates are washed 3 times with 100 µl/well PBST (0.05% Tween-20) on a BIOTEK plate washer. Plates are subsequently blocked for about 1 hour at room temperature with 2 % BSA in PBS. The plates are washed 3 times with 100 µl/well PBST (0.05%Tween-20) on the BIOTEK plate washer. Cell lysates (50µl) or standards (pEGFR pY1068 ELISA kit, R&D Systems, cat# DYC3570) diluted in 50% Lysis buffer and 1%BSA-PBS (per the manufacturer's recommendations) are added to the plates in duplicates and incubated for 2 hrs at room temperature or overnight at 4°C with shaking. Plates are then washed 3 times with 100 µl/well in the BIOTEK plate washer with PBST (PBS with 0.05%Tween-20). About 50 µl of a detection antibody (pEGFR pY1068 ELISA kit, R&D Systems, cat# DYC3570) conjugated to horseradish peroxidase (HRP) diluted (as per manufacturer's instructions) in 2% BSA, PBS is added and incubated for about 2 hour at room temperature. The plate is washed 3 times with 100 µl/well in the BIOTEK plate washer with PBST (0.05%Tween-20). About 50 µL of SUPERSIGNAL PICO ELISA substrate is added and the plate is read using an Envision (Perkin Elmer) plate reader. The data are analyzed and duplicate samples are averaged and error bars are used to represent the standard deviation between the two replicates.

The phospho-ERK ELISA is performed similarly to the phospho-EGFR ELISA with the following changes: Human pERK ELISA DUOSET kit is purchased from R&D Systems (cat# DYC1018-5) and used as recommended by the manufacturer.

The data are analyzed by subtracting background signal, regressing to a recombinant standard supplied by the manufacturer, and back-calculating the data (BCD) to correct for dilution factors. Duplicate samples are averaged and error bars are used when indicated to represent the standard deviation between two replicates.

### Example 1: Phospho-EGF Receptor and Phospho-ERK Signaling Inhibition by Single and Pairwise Combinations of Bin 1 + Bin 2 or Bin 1 + Bin 3 Antibodies and Comparisons with Each of Individual Monoclonal Antibodies Cetuximab, Nimotuzumab, and Zalutumumab.

A431 cells were treated with single antibodies or antibody pairs and their ability to inhibit EGFR-dependent signaling was compared to that each of cetuximab, nimotuzumab, and zalutumumab. Cells were incubated with varying concentrations of anti-EGFR antibodies for 2 hrs, and then stimulated with an EGFR ligand for 10 minutes at 37°C and 5% carbon dioxide. The seven recombinant human EGFR ligands used individually were 100ng/ml amphiregulin ("AREG," R&D Systems, cat # 262-AR/CF), 100ng/ml betacellulin (R&D Systems, cat # 261-CE-050/CF), EGF (PreproTech, cat # AF-100-15), 220ng/ml epigen (epithelial mitogen homolog, PreproTech, cat # 100-51), 150ng/ml epiregulin (R&D Systems, cat # 1195-EP/CF), 90ng/ml HB-EGF (heparin-binding EGF-like growth factor, PreproTech, cat # 100-47), and 50ng/ml TGFα (transforming growth factor alpha, R&D Systems, cat # 239-A). ELISA measurements were performed as described above for pERK and pEGFR signaling and the results are shown in Figures 1A-C. Only mixtures of Bin1/Bin2 antibodies cb and cd (Figure 1A) and Bin1/Bin3 antibodies cb and ch (Figure 1C) were effective at completely inhibiting phospho-ERK signaling when compared to cetuximab, nimotuzumab, and zalutumumab, as well as to individual components cb, cd, and ch. All antibodies, including the mixtures, were effective at complete inhibition of Phospho-EGF receptor signaling, with the exception of nimotuzumab (Figure 1B).

### Example 2: Phospho-ERK signaling inhibition by single and pairwise combinations of Bin 1, Bin 2, and Bin 3 antibodies and comparisons with cetuximab, nimotuzumab, and zalutumumab.

Single antibodies cb, cd, and ch, or pairs of cb and cd or cb and ch, (as described above in Example 1) were used to treat A431 cells at indicated total concentrations, and their ability to inhibit EGFR ligand-dependent signaling was compared to that of each single anti-EGFR antibodies cetuximab, nimotuzumab, and zalutumumab at the same concentrations. Cells were incubated with antibody for 2 hours followed by stimulation with EGFR ligand for 10 minutes. Seven EGFR ligands were used individually: amphiregulin (100ng/ml), betacellulin (100ng/ml), EGF (50ng/ml), epigen (220ng/ml), epiregulin (150ng/ml), HB-EGF (90ng/ml), and TGFα (50ng/ml). Experiments were performed as described above and the results are shown in Figures 2A-G and 3A-G. Individually, cb and cd, as well as well cetuximab, nimotuzumab, and zalutumumab, were effective at inhibiting phospho-ERK signaling (i.e., inhibiting phosphorylation of ERK1 and ERK2) in response to the three ligands with low affinity for EGF receptor (amphiregulin, epigen, and epiregulin), but not in response to the four ligands with high affinity for EGF receptor (betacellulin, EGF, HB-EGF, and TGFα). Only oligoclonal mixtures of Bin1/Bin2 antibodies cb and cd (Figures 2A-G) and Bin1/Bin3 antibodies cb and ch (Figures 3A-G) were effective at essentially completely inhibiting phospho-ERK signaling in response to all seven (both high- and low-affinity) EGFR ligands when compared to individual components of the mixtures, cb, cd, and ch and the other tested individual monoclonal antibodies, cetuximab, nimotuzumab, and zalutumumab.

### Example 3: Effect of EGFR ligand concentration on phospho-ERK cell signaling

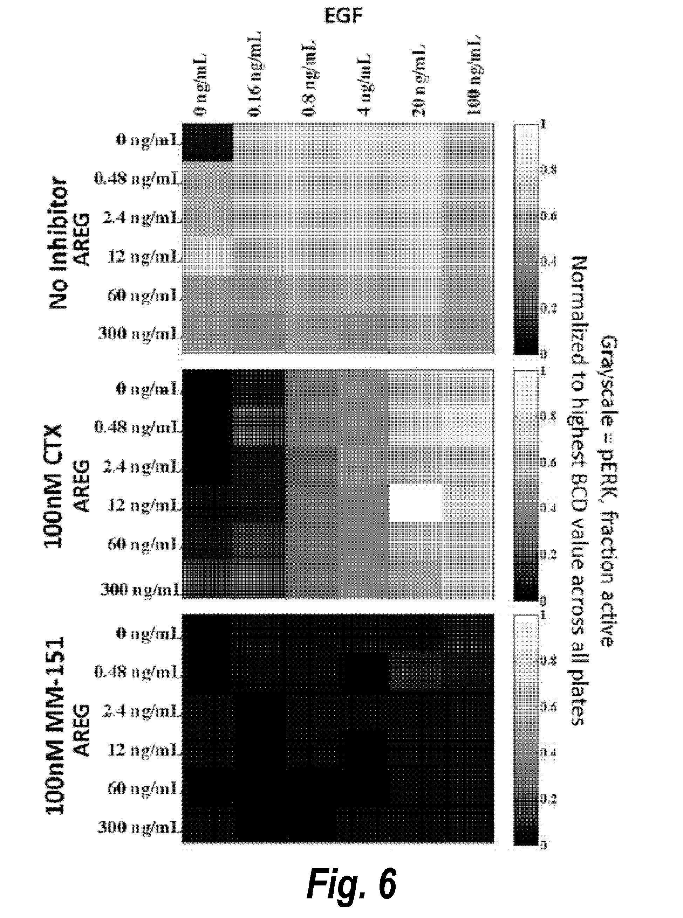

Inhibition of tumor cell signaling *in vitro* is analyzed by the methods described above or minor variations thereof. The epidermoid cancer cell line A431 was treated with media alone ("No Inhibitor"), MM-151 (100nM) or cetuximab (100nM) for 2hrs, followed by the addition of various concentrations of EGF (0.16ng/ml, 0.8ng/ml, 4.0ng/ml, 20ng/ml, 100ng/ml) or AREG (0.48ng/ml, 2.4ng/ml, 12ng/ml, 60ng/ml, 300ng/ml), alone or in combination, as shown in Figure 6. Cells were incubated with the various EGF and AREG ligand combinations for 10 minutes, lysed, and levels of ERK phosphorylation measured by phospho-ERK ELISA and analyzed as indicated in the methods. Figure 6 shows MM-151 and cetuximab-mediated modulation of ERK signaling represented as fraction of the highest signal across all treatments. Phospho-ERK signaling is inhibited by cetuximab in A431 cells under low-affinity EGFR ligand (AREG) stimulation, but become increasingly resistant to inhibition upon the addition of increasing amounts of the high-affinity EGFR ligand, EGF (middle panel), while inhibition of signaling by MM-151 is largely maintained under all conditions (lower panel).

### Example 4: Inhibition of Tumor Cell Proliferation in the Presence of High or Low Affinity EGFR Ligands

### Inhibition of Tumor Cell Proliferation In Vitro

Inhibition of cellular proliferation of cells expressing EGFR is examined *in vitro* as follows: H322M (NCI, Frederick, MD 21701), H1975 (ATCC CRL-2868™), and LIM1215 (Cell Bank Australia, NSW 2145) cancer cells are separately seeded in 96 well tissue culture plates at 5,000 cells per well and grown in RPMI-1640 medium supplemented with antibiotics, 2mM L-glutamine and 10% fetal calf serum (FCS) (H322M and H1975) or RPMI-1640 medium supplemented with 25mM HEPES, antibiotics, 2mM L-glutamine, 10% FCS, 0.6µg/ml insulin, 1µg/ml hydrocortisone and 10µM thioglycerol (LIM1215) for 24 hours at 37 degrees Celsius and 5% carbon dioxide. Medium is then switched to RPMI-1640 with antibiotics, 2mM L-glutamine, 1% FBS (for H322M and H1975) or RPMI-1640 with 25mM HEPES, antibiotics, 2mM L-glutamine, 1% FCS, 0.6µg/ml Insulin, 1µg/ml hydrocortisone and 10µM thioglycerol (for LIM1215) supplemented with 200ng/ml AREG, 50ng/ml EGF, 50ng/ml TGFα or 90ng/ml HB-EGF in the presence of varying concentrations of MM-151 or cetuximab (Bristol-Myers Squibb). Cell viability is measured 72 hours post-treatment using the CellTiter-Glo® (CTG) Luminescent Viable Cell Number Assay (Promega Corporation) according to manufacturer's instructions. The CTG assay measures the number of viable cells in culture based upon quantitation of ATP present, which is an indicator of metabolically active cells. Control treatments include cells treated with 1% FCS-containing medium (as detailed above) in the presence ("+Lig") or absence ("-Lig") of the respective ligand treatment. Viable cell numbers are plotted in GraphPad Prism (GraphPad Software, La Jolla, CA) as a fraction of the respective ligand ("+Lig") treatment control.

### Results

The non-small cell lung cancer (NSCLC) lines H322M and H1975 and colon cancer cell line LIM1215 were treated with varying concentrations of MM-151 or cetuximab (0.1-1 µM final concentration). Potent inhibition of growth of H322M, H1975 and LIM1215 cells was obtained over a range of MM-151 concentrations in the presence of high affinity EGFR ligands (EGF, TGFα, HB-EGF), but not in the presence of cetuximab or in assay medium alone (1 % FCS) -- Figure 4 (B-D, F-H, and J-L). Potent inhibition of growth of H322M, H1975 and LIM1215 cells was obtained over a range of concentrations for both MM-151 and cetuximab, but not by assay medium alone (1 % FCS) in the presence of the low affinity EGFR ligand amphiregulin (AREG) -- Figure 4 (A, E and I). These data demonstrate the ability of the MM-151 oligoclonal mixture to inhibit tumor cell proliferation *in vitro* in response to both high-affinity (EGF, TGFα, HB-EGF) and low-affinity (AREG) ligands, whereas cetuximab is only potently effective in cells treated with low-affinity (AREG) ligand.

### Example 5: Effects of EGF Ligand Concentration On Cell Proliferation

Using methods essentially as described in the preceding Example, non-small cell lung cancer (NSCLC) cell lines H322M, HCC827 and H1975 were treated with AREG alone (200ng/ml) or with AREG plus increasing amounts of EGF (0.2, 2, 20ng/ml) in the presence of varying concentrations of MM-151 or cetuximab (0.1-1µM final concentration).

### Results

The NSCLC cell lines respond to cetuximab under low-affinity EGFR ligand stimulation (AREG), but become increasingly unresponsive to treatment upon the addition of increasing amounts of the high-affinity EGFR ligand EGF, while sensitivity to MM-151 is largely maintained (see Figures 5A-5L).

### Example 6: Assays and Kits

### Measurement of EGFR Family Ligand Expression Levels by RT-qPCR

Measurement of EGFR ligand expression in tumor biopsy samples by real-time quantitative polymerase chain reaction (RT-qPCR) of DNAs reverse transcribed from RNAs is carried out as follows:

Total RNA is isolated from patient biopsy/tumor samples, *e*.*g*., by commercially available standard methods. The method of total RNA isolation may be any method (including conventional methods) suitable for use with the type of patient biopsy sample being tested, *e*.*g*., fresh, fixed, frozen, formalin fixed paraffin embedded (FFPE), etc. Total RNA is then converted to cDNA using the gene specific primers described below and Qiagen® OneStep RT-PCR reagents and protocol (Cat. #210210, Qiagen, Germantown, MD). The cDNA is then used for RT-qPCR using the following gene specific primers as TaqMan® probe sets obtained from Applied Biosystems (Carlsbad, CA) along with reagents and equipment from the same source, all as described below:
1. TaqMan® Gene Expression Assay, Gene Name: betacellulin, Assay ID: Hs01101201_m1
2. TaqMan® Gene Expression Assay, Gene Name: transforming growth factor, alpha, Assay ID: Hs00608187_m1
3. TaqMan® Gene Expression Assay, Gene Name: heparin-binding EGF-like growth factor, Assay ID: Hs00181813_ml
4. TaqMan® Gene Expression Assay, Gene Name: epiregulin, Assay ID: Hs00914313_m1
5. TaqMan® Gene Expression Assay, Gene Name: amphiregulin, Assay ID: Hs00950669_m1
6. TaqMan® Gene Expression Assay, Gene Name: epidermal growth factor, Assay ID: Hs01099999_m1.
7. TaqMan® Gene Expression Assay, Gene Name: epithelial mitogen homolog (epigen), Assay ID Hs02385425_ml.

5µl of diluted cDNA is mixed with 10µl of TaqMan® Fast Advanced Master Mix (Cat. # 4444556), 2µl of the above primer probe set and 3µl of water in a MicroAmp® Fast Optical 96-Well Reaction Plate (Cat. # 4366932). The plate is then placed in a Viia™ 7 RT-qPCR machine and a thermal cycling program completed as described in the manufacturers protocol. Data collection and analysis is carried out using the Viia™ 7-RUO-Software (Applied Biosystems).

Also see US Patent Publication Nos. 20030165952, 20040009489, 20050095634, 20050266420, 20070141587, 20070141588, 20070141589, 20080182255, 20090125247, 20090280490, 20100221754 and 20110086349, and US Patent Nos. 6,750,013, 6,808,888, 6,939,670, 6,964,850, 6,692,916, 7,081,340, 7,171,311, 7,526,387, 7,569,345, 7,622,251, 7,871,769, 7,838,224, 7,858,304, 7,930,104, and 8,071,286.

### Equivalents

Those skilled in the art will recognize, or be able to ascertain using no more than routine experimentation, many equivalents of the specific embodiments described herein. Such equivalents are intended to be encompassed by the following claims. Any combination of the embodiments disclosed in the any plurality of the dependent claims is contemplated to be within the scope of the disclosure.

## Claims

1. A method for determining whether a patient having a tumor is predicted to have a favorable outcome, comprising reduction of growth of the tumor, as a result of treatment (i) with a monoclonal anti-EGFR antibody preparation comprising a single species of monoclonal antibody; and/or as a result of treatment (ii) with an oligoclonal anti-EGFR antibody preparation comprising a plurality of species of monoclonal anti-EGFR antibodies, wherein the plurality of species of monoclonal anti-EGFR antibodies comprises at least two monoclonal antibodies that bind to two different extracellular epitopes of EGFR,
the method comprising:
(a) measuring levels at least two low affinity EGFR ligands selected from amphiregulin, epigen, or epiregulin in a biopsy sample of the tumor;
(b) measuring levels of at least two high affinity EGFR ligands selected from betacellulin, EGF, HB-EGF or TGFα in the biopsy sample; and
(c) comparing the average level of each of the high affinity EGFR ligands measured in (a) to the average level of each of the low affinity EGFR ligands measured in (b);
wherein,
if the average level of low affinity EGFR ligands measured in (a) is greater than the average level of high affinity EGFR ligands measured in (b), the patient is predicted to have a favorable outcome as a result of treatment (i) and the patient is predicted to have a favorable outcome as a result of treatment (ii); or
if the average level of low affinity EGFR ligands measured in (a) is less than or equal to the average level of high affinity EGFR ligands measured in (b), the patient is predicted to have an unfavorable outcome from treatment (i) and is predicted to have a favorable outcome from treatment (ii).

2. The method of claim 1, wherein levels of the EGFR ligands are determined by measuring levels of mRNA coding for the EGFR ligands.

3. The method of claim 2, wherein the levels of mRNAs are determined by reverse transcript PCR.

4. The method of any one of claims 1 to 3, wherein:
(a) the levels of three low affinity EGFR ligands are measured; and/or
(b) the levels of three or four high affinity ligands are measured.

5. The method of any one of the preceding claims, wherein the tumor is malignant.

6. The method of any one of the preceding claims, wherein the tumor is a tumor of the skin, central nervous system, head, neck, esophagus, stomach, colon, rectum, anus, liver, pancreas, bile duct, gallbladder, lung, breast, ovary, uterus, cervix, vagina, testis, germ cells, prostate, kidney, ureter, urinary bladder, adrenal, pituitary, thyroid, bone, muscle or connective tissue.

7. A kit for testing a tumor biopsy sample to determine levels of both high and low affinity EGFR ligands in the sample, which kit comprises one or more containers comprising:
(a) at least two pairs of high affinity EGFR ligand-specific polymerase chain reaction (PCR) primers, which are specific for at least two different high affinity EGFR ligands optionally selected from betacellulin, EGF, HB-EGF and TGFα;
(b) at least two pairs of low affinity EGFR ligand-specific PCR primers, which are specific for at least two different low affinity EGFR ligands optionally selected from amphiregulin, epigen and epiregulin; and
(c) at least one reverse transcription PCR (RT-PCR) reagent, such as one or more of a fluorescent reporter molecule suitable for use in a real-time RT-PCR assay, an RNA-dependent DNA polymerase, a DNA-dependent DNA polymerase and a solution comprising at least micromolar concentrations of each of deoxyadenosine triphosphate, deoxyguanosine triphosphate, deoxycytidine triphosphate, and deoxythymidine triphosphate.

8. The kit of claim 7, comprising pairs of high affinity EGFR ligand-specific primers specific to each of betacellulin, EGF, HB-EGF and TGFα and pairs of low affinity EGFR ligand-specific primers specific to each of amphiregulin, epigen and epiregulin.

9. The kit of claim 7 or 8, wherein the contents of all, or at least one of, the containers have been prepared under cGMP conditions and/or the one or more containers comprise either or both of:
(a) at least one container that has an internal temperature of below 20° C and above 0° C; and
(b) at least one container that has an internal temperature of below 0° C.

10. A monoclonal anti-EGFR antibody preparation comprising a single species of monoclonal antibody for use in the treatment of a tumor predicted by the method of any one of claims 1 to 6 to have a favorable outcome from treatment with the monoclonal anti-EGFR antibody preparation.

11. The preparation of claim 10 for use according to claim 10, wherein the monoclonal anti-EGFR antibody preparation comprises cetuximab, panitumumab, zalutumumab, matuzumab, or nimotuzumab.

12. An oligoclonal anti-EGFR antibody preparation comprising a plurality of species of monoclonal anti-EGFR antibodies, wherein the plurality of species of monoclonal anti-EGFR antibodies comprises at least two monoclonal antibodies that bind to two different extracellular epitopes of EGFR, for use in the treatment of a tumor predicted by the method of any one of claims 1 to 6 to have a favorable outcome from treatment with the oligoclonal anti-EGFR antibody preparation.

13. The preparation of claim 12 for use according to claim 12, wherein the plurality of species of monoclonal anti-EGFR antibodies comprises an antibody which inhibits binding of EGF to EGFR.

14. The preparation of claim 12 or 13 for use according to claim 12, wherein the oligoclonal anti-EGFR antibody preparation comprises three different species of monoclonal anti-EGFR antibodies.

15. The preparation of any one of claims 12 to 14 for use according to claim 12, wherein the oligoclonal anti-EGFR antibody preparation comprises:
(a) a monoclonal anti-EGFR antibody comprising the V_{H} sequence of SEQ ID NO:1 and the V_{L} sequence of SEQ ID N0:2;
(b) a monoclonal anti-EGFR antibody comprising the V_{H} sequence of SEQ ID NO:3 and the V_{L} sequence of SEQ ID N0:4; and
(c) a monoclonal anti-EGFR antibody comprising the V_{H} sequence of SEQ ID NO:5 and the V_{L} sequence of SEQ ID N0:6.
